(19) Europäisches Patentamt
European Patent Office
Office européen des brevets

(11) **EP 2 046 251 B1**

## (12) EUROPEAN PATENT SPECIFICATION

(45) Date of publication and mention
of the grant of the patent:
**06.10.2010 Bulletin 2010/40**

(21) Application number: **07787994.8**

(22) Date of filing: **27.07.2007**

(51) Int Cl.:
**A61F 9/01** (2006.01)

(86) International application number:
**PCT/EP2007/057780**

(87) International publication number:
**WO 2008/015174 (07.02.2008 Gazette 2008/06)**

(54) **METHOD AND APPARATUS FOR CALCULATING A LASER SHOT FILE FOR USE IN A REFRACTIVE EXCIMER LASER**

VERFAHREN UND GERÄT ZUR BERECHNUNG EINER LASERSCHUSSDATEI ZUR VERWENDUNG IN EINEM REFRAKTIVEN EXCIMER-LASER

PROCÉDÉ ET APPAREIL POUR CALCULER UN FICHIER DE TIR LASER À UTILISER DANS UN LASER À EXCIMÈRE REFRACTIF

(84) Designated Contracting States:
**DE ES FR GB IT**

(30) Priority: **02.08.2006 DE 102006036086**

(43) Date of publication of application:
**15.04.2009 Bulletin 2009/16**

(73) Proprietor: **Technolas Perfect Vision GmbH
80992 München (DE)**

(72) Inventor: **HEGELS, Ernst
85551 Kirchheim (DE)**

(74) Representative: **Schmidt, Josef Heinrich
Vossius & Partner
Siebertstrasse 4
81675 München (DE)**

(56) References cited:
**US-A- 6 132 424     US-A1- 2003 023 233
US-B1- 6 635 051**

**Description**

Field of the invention

The present invention relates to a method and apparatus for calculating a laser shot file for use in a refractive excimer laser in particular using a dithering algorithm. The invention is specifically suitable for applying the laser shot file when performing a laser treatment of an eye or when producing a customized contact lens or an intraocular lens (IOL) by laser ablation.

Description of the related art

US 6,090,100 relates to an excimer laser system for correction of vision with reduced thermal effects. It specifically relates to an apparatus and method for controlling the excimer laser system for removing tissue from the eye to perform various types of corrections, such as myopia, hyperopia, and astigmatism correction. In one disclosed embodiment, the excimer laser system provides a relatively large spot size which provides a relatively large coverage of treatment area per shot. While using such large spot sizes, the shots are generally not "adjacent" to each other but instead overlap to generate the desired degree of ablation at a particular point. For calculating the result of the overlapping shots, an algorithm is used. In one method of calculating treatment patterns using large, fixed spot sizes distributed throughout the treatment area, a dithering algorithm is used. Specific reference is made to a rectangular dithering, circular dithering and a line-by-line oriented dithering. Using any variety of shot dithering methods, an array of shots is created for a fixed spot size spread over a treatment area to correct to the desired degree of ablation. For the respective array, a grid is used with a constant grid width between individual grid positions. With the known dither methods, the shape of the desired ablation profile, which usually is a continuous profile, has to be transferred into a whole-numbered discrete density distribution. Here, the continuous profile represents a planned ablation and the whole-numbered discrete density distribution represents a series of ablating flying spot laser pulses. The residual structure, i.e., the difference between the planned and the achieved profile, has to be minimised. Exact solutions can principally be found numerically but not in a reasonable time. Therefore, for this purpose, dither algorithms are used. The profile is discretised on a given grid. Using a cost function or merit function the algorithm decides for each position of the grid whether to place a shot or not. For this decision, usually only a few neighbouring positions of the grid are taken into account. This dither algorithm saves calculation time without the need that the real size of the spot is taken into account. It is sufficient to know the shot volume which is ablated with one laser shot. However, under certain conditions, the known dither algorithms produce artefacts in parts of the profile, e.g., in low-density regions where the next neighbouring shot is too far away. Artefacts may also be produced in high-density regions where at nearly every position, a shot is placed. The positions with no shot also have too large a distance for the assumption that only a few neighbour positions are necessary.

As regards the general background of dithering algorithms, reference is made to US 6,271,936 B1, which relates to the field of digital image processing. It particularly relates to a method for digitally multitoning a continous-tone image using error diffusion, dithering and over-modulation methods. Reference is made to the problem that an artefact may occur like worms which are formed when the black or white output pixels appear to string together in an area that should be otherwise uniform. Wherein this US patent gives a detailed description of these known methods, it is related to a completely different technical field. Among other differences, known laser printer systems are using a respective fixed resolution given as a number of dots per inch, i.e., a higher number of dots per inch results in a better resolution. Moreover, a known laser printer has no problem with overlapping and touching dots because this does not result in an additional blackening when hitting a point twice or more often. Rather, to produce an image, a certain local area of the image having a certain grey level can be created by applying a corresponding number of dots in this local area.

Finally, US 2003/023233 discloses the determination of a grid width, but this grid with is not based upon the calculated shot density and no dithering algorithm is used.

Summary of the invention

The object underlying the present invention is to provide a method and apparatus for calculating a laser shot file for use in a refractive excimer laser, wherein the difference between the planned and the achieved profile is minimised. This object is solved with the features of claims 1 and 11.

A desired ablation profile for correcting for example myopia has a maximum shot density in the central part of the treatment zone whereas a minimum shot density is present along the circumferential border of the treatment zone. Thus, the number of laser shots to be applied to the central part of the treatment zone is higher than in other sub-areas in particular along the border of the treatment zone.

For the correction of, for example, hyperopia the minimum shot density is present in the central part of the treatment zone. On the other hand, the ablation profile requires a higher number of laser shots along a circumferential border of the treatment zone.

The invention is generally applicable for any ablation profile, wherein sub-areas having different shot densities are investigated in order to determine any sub-area having a maximum shot density and/or any sub-area having a minimum shot density.

The general concept of the present invention is based on the idea to optimise the grid, in particular to optimise the grid width of a grid which is used for placing laser shots of the excimer laser. More specifically, the shot density for obtaining a predetermined desired ablation profile is calculated first. Depending on the calculated shot density of the desired ablation profile, an optimum grid, i.e., an optimum grid width is determined.

According to a preferred embodiment of the present invention, the grid width is optimised depending on the minimum shot density and/or maximum shot density of the desired ablation profile. Generally, for a desired ablation profile having low shot densities, a grid having a wider grid width is used. For a desired ablation profile having high shot densities, a grid having a narrow grid width is used. Preferably, one grid width is selected which fulfils the requirement that the minimum number of occupied grid positions is at least 4% of all available grid positions in any region and/or the maximum number of occupied grid positions is not more than 96% of all available grid positions in any sub-area of a treatment zone. An occupied grid position preferably receives only one laser shot. Preferable, the range is 10% to 90% and most preferably the range is 20% to 80%.

The grid width is preferably at least a value within the range of $10\mu$m to $300\mu$m and preferably within the range of $30\mu$m to $240\mu$m.

According to a preferred embodiment, a dither algorithm is used for calculating the placement of the laser shots of the excimer laser on grid positions. The dither algorithm is adapted to the desired ablation profile by determining the optimised grid width for the grid to be used for the dither algorithm.

A local shot density $D(x, y)$ within a sub-area around a grid position $P(x, y)$ is calculated from an ablation profile $z(x, y)$ within the respective sub-area using the ablation volume of a single laser shot $V_{Shot}$ and a grid width G using following equation:

$$D(x, y) = z(x, y) * G^2 / V_{Shot} \qquad (1)$$

With following equation the grid width is found for a maximum value of the Profile $z_{max}(x, y)$ and for a desired maximum density $D_{max}(x, y)$:

$$G = \sqrt{V_{Shot} * D_{max}(x, y) / z_{max}(x, y)} \qquad (2)$$

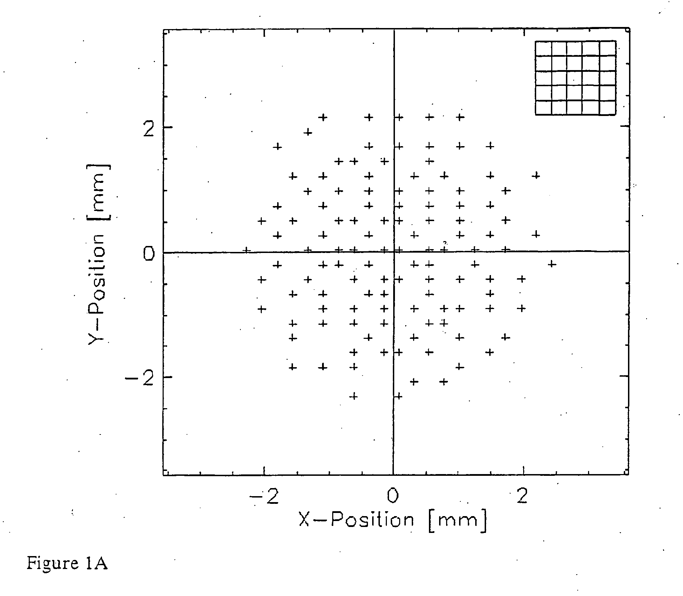

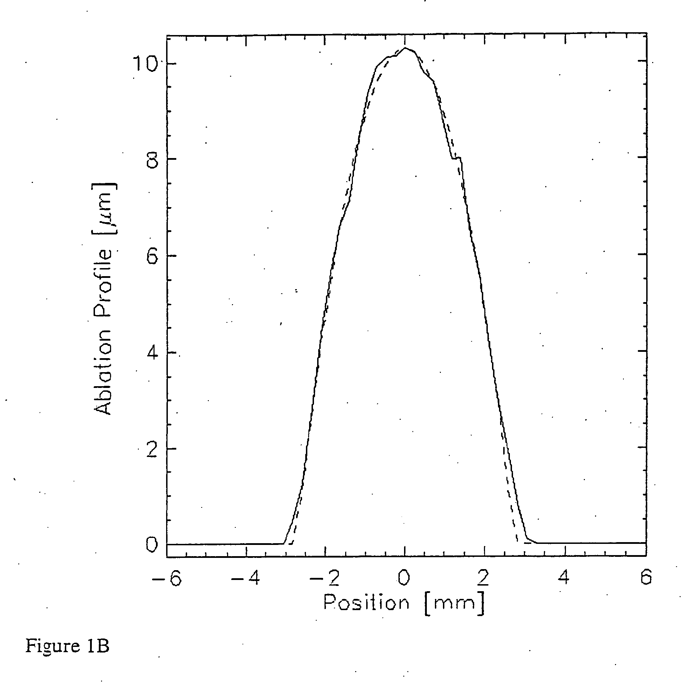

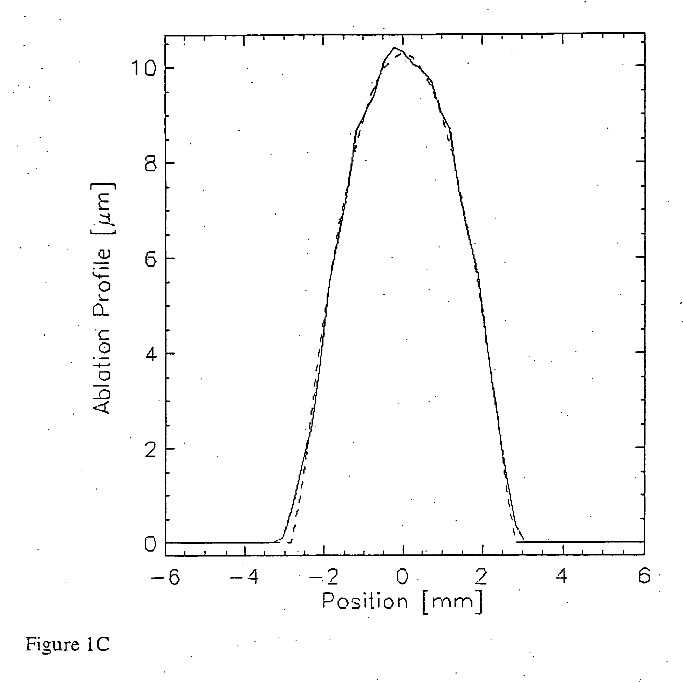

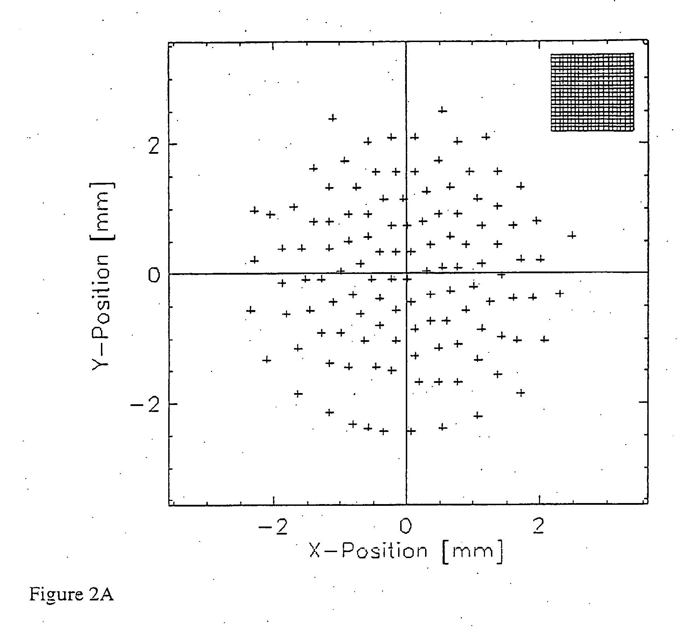

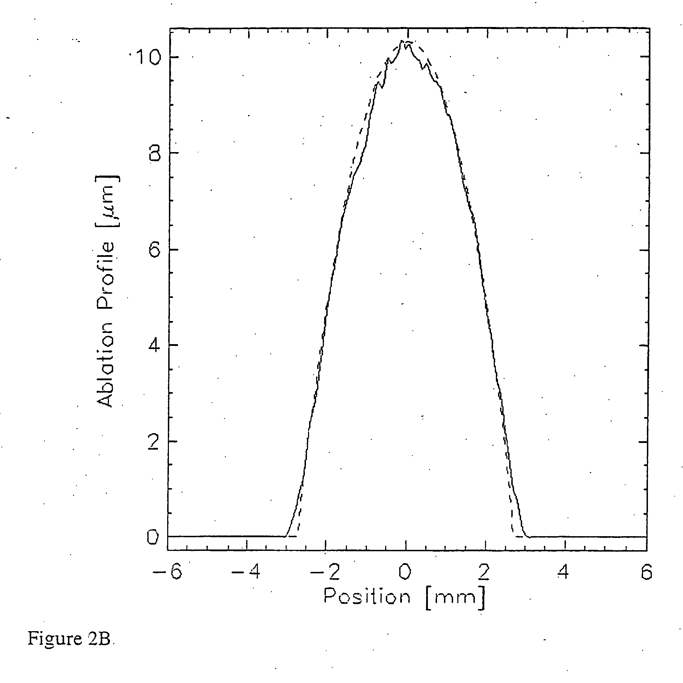

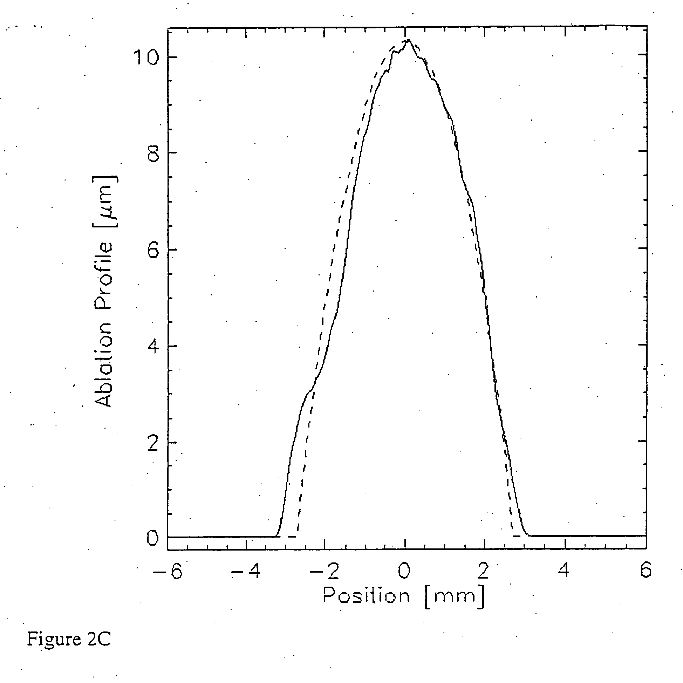

With equation 1 the local shot density around the minimum of the desired profile is calculated with the grid width of equation 2. The influence of the grid width is explained using two examples. As a first example a treatment using a treatment zone of about 5mm for a desired correction of -1dpt is selected. This myop correction has the maximum of the ablation in its centre. The desired depth is approximately $10\mu$m. About 120 laser shots are necessary to reach a result with a typical excimer treatment laser. To get shot densities in the central part of about 54% a grid constant of 235 $\mu$m is chosen (Figure 1). With a grid constant of 59 $\mu$m the shot density in the central part is only 3.3%., which is the cause of deviations of the result compared with the desired ablation (Figure 2). In a second example of an ablation the treatment zone is 7.5mm and the correction is -8dpt. The desired maximum depth is about $160\mu$m and about 4000 laser shots are needed. For this example, the grid constant of $59\mu$m comprises good results. The maximum shot density is 54.5%. These two examples show the advantage to calculate the grid constant for each profile. The given shot numbers and refractive correction depend on the laser shot energy. Here a typical laser energy is assumed.

If the minimum density threshold is hurt, the profile is preferably split into at least two sub-protiles.

According to a further preferred embodiment, a desired ablation profile is divided into at least two ablation sub-profiles. Then for each ablation sub-profile, the respective shot density is calculated and a respective grid width based on the respective calculated density of the ablation sub-profile is determined. Thus, for a desired ablation profile where the contrast is too high, i.e., a difference between the maximum shot density and the minimum shot density is too high, the calculation of the laser shot file is made in two or more runs preferably using different grid constants or grid widths for each respective ablation sub-profile resulting in a corresponding laser shot file. Thereafter, the two or more laser shot files can be combined in one single laser shot file.

According to the present invention, the calculated, placed laser shots are processed in a further step of sorting to obtain a laser shot sequence. The sorting is performed taking into consideration that any thermal effects should be avoided, i.e., two consecutive laser shots are preferably placed on different grid positions in the treatment zone which are at a distance from each other.

<u>Brief description of the drawings</u>

The invention will be further described by way of examples with reference to the drawings, in which:

Fig. 1A is a diagram showing the location of laser spots for a first test using a first grid width,
Fig. 1B is a diagram showing the planned and the achieved profile as a cross-section along the horizontal axis of Fig. 1A,
Fig. 1C is a diagram showing the planned and the achieved profile as a cross-section along the vertical axis of Fig. 1A,
Fig. 2A is a diagram showing the location of laser spots for a second test using a second grid width,
Fig. 2B is a diagram showing the planned and the achieved profile as a cross-section along the horizontal axis of Fig. 2A,
Fig. 2C is a diagram showing the planned and the achieved profile as a cross-section along the vertical axis of Fig. 2A,
Fig. 3 shows a flow diagram with a calculation of laser pulse patterns with a dither algorithm, and
Fig. 4 shows an example of a sub-grid with weighting factors usable for weighting neighbouring error values.

<u>Detailed description of the preferred embodiment</u>

Figs. 1A, 1B and 1C show the simulated calculation of a laser shot file for use in a refractive excimer laser for the correction of myopia with a value of about -1 dioptres, using a typical excimer laser for refractive treatments, within a treatment zone having a diameter of 5.5 mm and using a laser spot having a diameter of I mm. In this simulated first test, the grid width is 235$\mu$m. Thus, the distance between two neighbour grid points is 235$\mu$m. In this example, the grid points are arranged in rows and columns. In total, 120 laser shots are used for achieving a small ablation. Depending on the ablated volume of a single shot the resulting treatment is expected to have a refraction of said about -1 dioptres. The diagram of Fig. 1A shows the respective centre position of each of the 120 laser shots which is related to one of the grid positions each marked with a "+"-sign. In the upper right corner of Fig. 1A, the grid is schematically shown having a grid width of 235$\mu$m. Each of the shown laser shot centre positions are arranged on a grid point of this grid. The diagram of Fig. 1B shows, as a dashed line, the desired ablation profile, i.e., the ablation depth in $\mu$m with respect to a respective X-position. The ablation depth is approximately 10$\mu$m in the central part of the treatment zone and is smaller to both sides. At the x-positions -3 and +3, the ablation depth is zero. It further shows the simulated resulting ablation profile as a continuous line as a cross-section taken along the horizontal axis through the point 0-0 in Fig. 1A. Similarly, Fig. 1C shows the desired ablation profile as a dashed line which is taken as a cross-section along the vertical axis through the point 0-0 in Fig. 1A. Fig. 1C further shows the resulting ablation profile as a continuous line taken as a cross-section along the vertical axis through point 0-0 of Fig. 1A. In Fig. 1 the average shot density inside the treatment zone, having in this example a diameter of 5.5mm, is about 27.7% (Figure 1A). The respective centre positions of the laser shots are placed within a range of $\pm$ 2.2mm in the X-direction and $\pm$ 2.2mm in the Y-direction. The maximum shot density is about 53,9% for the central part of the treatment zone.

Figs. 2A, 2B and 2C show the results of a similar second test as in Figs. 1A, 1B and 1C except for a different grid width of 59$\mu$m. Thus, the grid width for the second test is about one fourth of the grid width for the first test. This has the effect that the number of grid points for the second test per unit area is about 16 times of the number of the grid points for the first test per unit area.

In Fig. 2 the average shot density inside the treatment zone, having in this example a diameter of 5.5mm, is about 1.7%. The maximum shot density is about 3.3% for the central part of the treatment zone. Thus, when comparing the average shot density for the first test which is about 27.7% and the average shot density of the second test which is about 1.7%, the resulting factor is 16. Similarly, the factor is 16 when comparing the maximum shot density of about 53.9% for the first test and the maximum shot density of about 3.3% for the second test. This clearly shows that the shot density can be adjusted by selecting an appropriate grid width. With other words, by selecting an appropriate number of available grid points in relation to the number of shots to be placed in the central part of the treatment zone a predetermined shot density can be achieved.

This low shot density for the second test causes artefacts like the sickle-shaped worms in the lower part of the ablation (Figure 2A). As shown, several laser shots are provided at grid positions which are arranged along a curved line at a closer distance. Further laser shots are provided at grid positions which are arranged at a larger distance from this curved line. Thus, the laser shots are not provided in an equal manner resulting in a deviation from the desired ablation profile (see Fig. 2C).

A comparison of the Figures for the first test and the second test shows that the resulting ablation profile in the first test is better, i.e., the curve of the resulting ablation profile better follows to the curve of the desired ablation profile (see Figs. 1B and 1C). In particular, Figs. 2B and 2C show that the resulting ablation profile deviates from the desired ablation profile, i.e., there is a shift with respect to the centre of the treatment zone and both curves comprise additional maxima and minima in the rising and the falling edge as shown in Fig. 2B and the rising edge as shown in Fig. 2C.

According to the present invention, in particular a grid width is determined such that within a treatment zone, a minimum number of grid positions receive one laser shot in sub-areas where the calculated shot density is low. Preferably, the minimum number of grid positions in sub-areas where the calculated shot density is low is at least 4% of the total number. In other words, at least one grid position of 25 grid positions in a particular sub-area of the treatment zone should receive one laser shot. Preferably, at least 10% of grid positions in sub-areas where the calculated shot density is low receive a laser shot. In other words, one grid position out of ten grid positions in a particular sub-area receive one laser shot. More preferably, at least 20% of grid positions in said sub-areas where the calculated density is low receive a laser shot. In other words, one out of five grid positions in said sub-areas receive a laser shot.

By using the same grid, i.e., with the same grid width, the maximum number of grid positions in regions where the calculated shot density is high is not more than 96%. In other words, one grid position out of 25 grid positions in sub-areas where the calculated density is high will not receive a laser shot. Preferably, not more than 90% of grid positions receive a laser shot, i.e., one out of ten grid positions does not receive a laser shot. More preferably, not more than 80% of grid positions in said sub-areas where the calculated density is high receive a laser shot, i.e., one out of five grid positions does not receive a laser shot.

The above ranges are determined to avoid any deviation from the resulting ablation profile from a desired ablation profile. More specifically if, e.g., on the one hand, laser shots are placed at every grid position in a region of the treatment zone, so called artefacts may be present. On the other hand, if laser shots are provided at too few grid positions, so called worms are present. Such artefacts and worms can be avoided by appropriately selecting the grid width of the grid.

As a general rule, the adjustment of the grid width has the following effect. On the one hand, if the grid width is made wider, e.g., by the factor of 2, the number of grid points will decrease to 1/4. On the other hand, if the grid width has changed to become narrower, e.g., by a factor of 2, the number of grid points will increase by the factor of 4.

By using a dither algorithm, the input parameters are the shot volume of a laser shot and the desired ablation profile. There is no need to take the beam diameter into account as the dither algorithm works independently therefrom. The dither algorithm provides a laser shot file as an output. More specifically, the dither algorithm is used for the placement of laser shots of the excimer laser on grid positions. Preferably, a cost function is used for deciding for each grid position whether a laser shot is placed or not. Herein, preferably the decision is made with regard to whether one or more laser shot(s) is (are) placed at a grid position(s) within the neighbourhood of the given grid position. Preferably, a dither algorithm is used as disclosed in US 6,090,100.

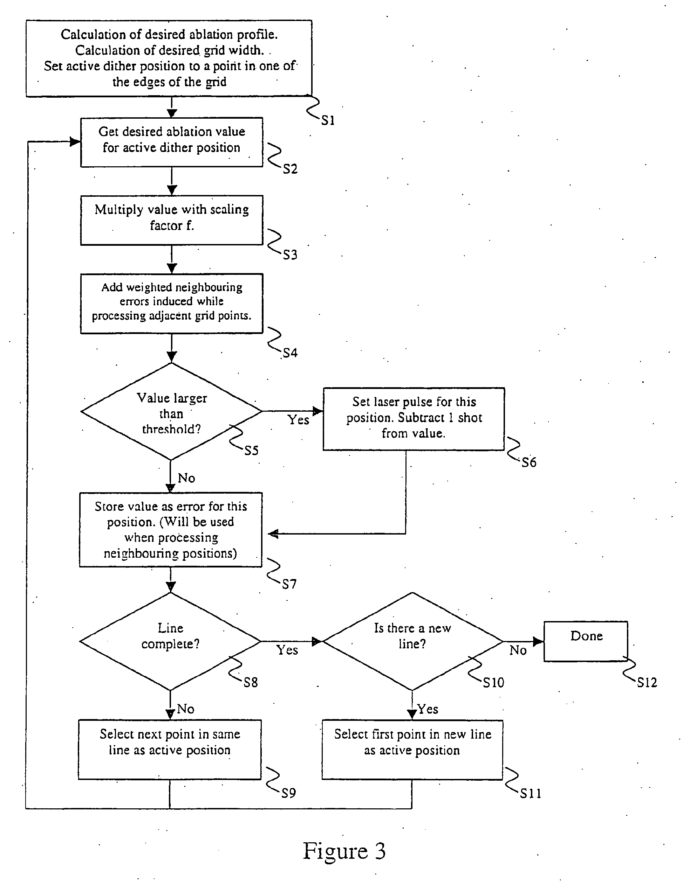

In the following a preferred dithering algorithm will be described with reference to Figure 3 which shows a flow chart representing an example for the error diffusion. This dither algorithm is based on the concept of error diffusion. Prior to the step of error diffusion, the desired ablation profile is calculated based, e.g., on the desired correction of a patient's eye or the modification of contact lenses or of IOLs. This profile is stored within a grid having a specific grid width. For example, such a grid has 256 x 256 values which covers an area of $15^2$ mm$^2$. The error diffusion may be started in one edge within that grid and follows it line by line.

In a first step S1, the ablation profile and the grid width is determined using equation (2) and the active dither position is set to a point in one of the edges of the grid. Said active dither position represents the actual position within the grid being processed.

In a next step S2, a desired ablation value for the active dither position is obtained. In step S3 this desired ablation value is multiplied with a scaling factor f. The scaling factor f takes into account the different size of laser pulse and the positioning step, i.e., the grid width. More specifically, the scaling factor is calculated as follows to get the desired shot density at this position (see equation 1):

$$f = \frac{(\text{Grid width})}{V_{shot}}$$

For the above-mentioned grid having 256 x 256 values covering an area of $15^2$ mm$^2$, the grid width is 15 mm/ 256 = 58 $\mu$m. Thus, the area of the smallest square the laser beam can be sent around is $(58 \, \mu m)^2$. Thus, the number of calculated pulses are reduced in order to take into account for the overlapping of laser pulses.

In a next step S4, weighted neighbouring errors are added to the scaled desired ablation value for the active dither position. These weighted neighbouring errors are preferably the weighted sum of errors of adjacent grid points that have already been processed. An example will be described later.

In a further step S5, a decision is made whether the obtained value is larger than a predetermined threshold. Thus, the sum of the value for the respective grid point and the weighted errors of adjacent grid points will be compared to this threshold value. If the value is not larger than the threshold step S9 follows. If the value is larger than the threshold, a laser pulse is set for this grid position in step S6. One laser pulse is subtracted from said density value.

On the other hand, if the new value is not larger than a threshold in step S7, this new value is stored as an error for this particular grid position. It will be used when processing neighbouring positions for the calculation with respect to further dither positions.

In the next step S8, it is decided whether the line is complete; if not, in step S9 a next point in the same line is selected as an active position and the before-mentioned processing is repeated. In case the line is complete, then in step S10 a decision has to be made whether there is a new line; if yes, then in step S11 a first point in the new line is selected as active position and the processing is repeated. Otherwise, if there is no new line, the processing ends with step S 12. The before-mentioned grid point error represents the ablation error done at a particular grid point. For each grid point processed, this error is the sum of desired ablation value plus the weighted neighbouring errors minus the laser pulse ablation depth (if a laser pulse has been set for that position).

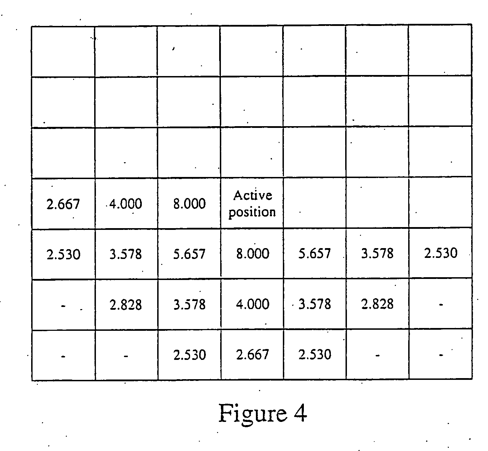

Figure 4 shows an example for weighting of errors of neighbouring grid points. More specifically, Figure 4 shows a sub-grid of 7 x 7 grid points, wherein the active dither position is shown in the middle. In this case, the weighting function is determined as 8/distance with a distance measured in units of grid points. The sum of the errors will then be normalised by a division with 70.736 which is the sum of all weighting factors used. As apparent from Figure 4, the white positions indicate grid position not yet processed. Thus, before deciding whether a laser pulse has to be set at a given grid position, the error induced while processing adjacent grid points has to be added to the theoretical ablation value for that grid point. The errors of the neighbouring grid points are not simply added but weighted due to their distance to the active grid point. The respective weighting factors are shown in Figure 4. It shall be noted that this is just one possible method for summing up the surrounding errors, which is working fine.

Tests have shown that the threshold value delivering good results was a positive value near 0. Preferably, a threshold value is used which corresponds to 1/2048.

It shall be noted that the above described dither algorithm is only one example for using the present invention.

A laser shot sequence may be determined thereafter by using a separate sorting algorithm. A sorting may be performed in order to avoid thermal effects. Thus, any two following laser shots should preferably be placed at two grid positions at a distance from each other.

Preferably, every four shots a laser shot is placed in the same region as the first shot.

The foregoing disclosure and description of the invention are illustrative and explanatory thereof and changes in the construction and method of operation may be made without departing from the scope of the invention.

**Claims**

1. A method for calculating a laser shot file for use in an excimer laser preferably for performing a refractive laser treatment of an eye or for producing a customized contact lens or an intraocular lens comprising the steps of using means for providing information with respect to a desired ablation profile, using means for calculating a shot density for obtaining the desired ablation profile wherein the shot density is the number of laser shots to be applied to a part of a treatment zone, using means for determining a grid width of a grid being used for placing laser shots of the excimer laser on grid positions wherein the grid width is the distance between two neighbour grid points of the grid and wherein the grid width is determined based on the calculated shot density of the desired ablation profile for calculating said laser shot file using a dithering algorithm, wherein the grid width is determined such that within a treatment zone a minimum number of grid positions receive one laser shot in sub-areas where the calculated shot density is low and/or such that a maximum number of grid positions receive one laser shot in sub-areas where the calculated shot density is high.

2. The method of claim 1, wherein within the treatment zone the minimum number of grid positions in sub-areas where the calculated shot density is low is at least 4 percent, preferably at :least 10 percent and more preferably at least 20 percent of grid positions in said sub-areas where the calculated shot density is low.

3. The method of claim 1 or 2, wherein within the treatment zone the maximum number of grid positions in sub-areas where the calculated shot density is high is not more than 96 percent, preferably not more than 90 percent and more preferably not more than 80 percent of grid positions in said sub-areas where the calculated shot density is high.

4. The method of any of the foregoing claims, wherein the grid width G is determined by using the equation:

$$G = \sqrt{V_{shot} * D_{max}(x, y) / z_{max}(x, y)} \, .$$

wherein

$V_{Shot}$ is the ablation volume of a single laser shot.

$D_{max}(x, y)$ is the local maximum shot density at a grid position P(x,y), $z_{max}(x, y)$ is the maximum value of the ablation profile at the grid position P(x,y).

5. The method of any of the foregoing claims further comprising the step of calculating the placement of the laser shots of the excimer laser on grid positions using said dither algorithm.

6. The method of claim 5 further comprising the step of deciding for each grid position whether to place a laser shot or not by using a cost function of the dither algorithm.

7. The method of claim 6 wherein in the step of deciding whether to place a shot on a given grid position a corresponding decision with regard to the grid positions in the neighborhood of the given grid position is taken into account.

8. The method of any of the foregoing claims further comprising the step of dividing a desired ablation profile into at least two ablation sub-profiles, calculating the shot density of each of said ablation sub-profiles, determining a respective grid width based on the respective calculated shot density of each of the ablation sub-profiles.

9. The method of any of claims 5 to 8 further comprising the step of sorting the calculate placed laser shots.

10. The method of any of the foregoing claims, wherein the refractive excimer laser provides a laser beam at a spot size fixed between 0.5 mm and 3.5 mm in diameter, preferably at a spot size fixed between 1.0 to 2.0 mm in diameter.

11. An apparatus for calculating a laser shot file for use in a refractive excimer laser preferably for performing a refractive laser treatment of an eye or for producing a customized contact lens or an intraocular lens comprising means for providing information with respect to a desired ablation profile, means for calculating a shot density for obtaining the desired ablation profile, wherein the shot density is the number of laser shots to be applied to a part of a treatment zone, means for determining a grid width of a grid being used for placing laser shots of the excimer laser on grid positions wherein the grid width is the distance between two neighbour grid points of the grid and wherein the grid width is determined based on the calculated shot density of the desired ablation profile for calculating said laser shot file using a dithering algorithm, wherein the grid width is determined such that within a treatment zone a minimum number of grid positions receive one laser shot in sub-areas where the calculated shot density is low and/or such that a maximum number of grid positions receive one laser shot in sub-areas where the calculated shot density is high.

12. The apparatus of claim 11, wherein within the treatment zone the minimum number of grid positions in sub-areas where the calculated shot density is low is at least 4 percent, preferably at least 10 percent and more preferably at least 20 percent of grid positions in said sub-areas where the calculated shot density is low.

13. The apparatus of claim 11 or 12, wherein within the treatment zone the maximum number of grid positions in sub-areas where the calculated shot density is high is not move than 96 percent; preferably not more than 90 percent and more preferably not more than 80 percent of grid positions in said sub-areas where the calculated shot density is high.

14. The apparatus of any of claims 11 to 13, wherein the grid width G is determined by using the equation

$$G = \sqrt{V_{Shot} * D_{max}(x, y) / z_{max}(x, y)},$$

wherein

$V_{Shot}$ is the ablation volume of a single laser shot,

$D_{max}(x, y)$ is the local maximum shot density at a grid position P(x,y),

$z_{max}(x, y)$ is the maximum value of the ablation profile at the grid position P(x,y).

15. The apparatus of any of claims 11 to 14 further comprising means for calculating the placement of the laser shots of the excimer laser on grid positions using said dither algorithm.

**16.** The apparatus of claim 15 further comprising means for deciding for each grid position whether to place a laser shot or not by using a cost function of the dither algorithm.

**17.** The apparatus of claim 16 wherein the means for deciding whether to place a shot on a given grid position receives information of a corresponding decision with regard to the grid positions in the neighborhood of the given grid position.

**18.** The apparatus of any of claims 11 to 17 further comprising means for dividing a desired ablation profile into at least two ablation sub-profiles, calculating the shot density of each of said ablation sub-profiles, wherein said means for determining a grid width receives the respective calculated shot density of each of the ablation sub-profiles and determines the respective grid width.

**19.** The apparatus of any of claims 15 to 18 further comprising means for sorting the calculated placed laser shots.

**20.** The apparatus of any of claims 11 to 19, wherein the refractive excimer laser provides a laser beam at a spot size fixed between 0.5 mm and 3.5 mm in diameter, preferably at a spot size fixed between 1.0 to 2.0 mm in diameter.


**Patentansprüche**

**1.** Verfahren zur Berechnung einer Laserschußdatei zur Verwendung in einem Excimer-Laser vorzugsweise zur Durchführung einer refraktiven Laserbehandlung eines Auges oder zur Herstellung einer kundenspezifischen Kontaktlinse oder einer Intraokularlinse, das die Schritte aufweist: Verwenden einer Einrichtung zum Bereitstellen von Informationen bezüglich eines gewünschten Ablationsprofils, Verwenden einer Einrichtung zur Berechnung einer Schußdichte zum Erhalten des gewünschten Ablationsprofils, wobei die Schußdichte die Anzahl der Laserschüsse ist, die auf einen Teil einer Behandlungszone angewendet werden soll, Verwenden einer Einrichtung zur Bestimmung einer Gitterweite eines Gitters, das zum Plazieren von Laserschüssen des Excimer-Lasers auf Gitterpositionen verwendet wird, wobei die Gitterweite der Abstand zwischen zwei Nachbargitterpunkten des Gitters ist und wobei die Gitterweite beruhend auf der berechneten Schußdichte des gewünschten Ablationsprofils bestimmt wird, zur Berechnung der Laserschußdatei mittels eines Dithering-Algorithmus, wobei die Gitterweite so bestimmt wird, daß innerhalb einer Behandlungszone eine minimale Anzahl von Gitterpositionen einen Laserschuß in Teilflächen empfängt, wo die berechnete Schußdichte niedrig ist, und/oder daß eine maximale Anzahl von Gitterpositionen einen Laserschuß in Teilflächen empfängt, wo die berechnete Schußdichte hoch ist.

**2.** Verfahren nach Anspruch 1, wobei innerhalb der Behandlungszone die minimale Anzahl von Gitterpositionen in Teilflächen, wo die berechnete Schußdichte niedrig ist, mindestens 4 Prozent, vorzugsweise mindestens 10 Prozent und besonders bevorzugt mindestens 20 Prozent der Gitterpositionen in den Teilflächen beträgt, wo die berechnete Schußdichte niedrig ist.

**3.** Verfahren nach Anspruch 1 oder 2, wobei innerhalb der Behandlungszone die maximale Anzahl von Gitterpositionen in Teilflächen, wo die berechnete Schußdichte hoch ist, nicht mehr als 96 Prozent, vorzugsweise nicht mehr als 90 Prozent und besonders bevorzugt nicht mehr als 80 Prozent der Gitterpositionen in den Teilflächen beträgt, wo die berechnete Schußdichte hoch ist.

**4.** Verfahren nach einem der vorhergehenden Ansprüche, wobei die Gitterweite G mittels der Gleichung bestimmt wird:

$$G = \sqrt{V_{Shot} * D_{max}(x, y) / z_{max}(x, y)} \ ,$$

wobei

$V_{Shot}$ das Ablationsvolumen eines einzelnen Laserschusses ist,
$D_{max}(x, y)$ die lokale maximale Schußdichte an einer Gitterposition P(x,y) ist,
$z_{max}(x, y)$ der Maximalwert des Ablationsprofils an der Gitterposition P(x,y) ist.

**5.** Verfahren nach einem der vorhergehenden Ansprüche, das ferner den Schritt des Berechnens der Plazierung der Laserschüsse des Excimer-Lasers auf Gitterpositionen mittels des Dithering-Algorithmus aufweist.

6. Verfahren nach Anspruch 5, das ferner den Schritt aufweist, für jede Gitterposition zu entscheiden, ob ein Laserschuß plaziert werden soll oder nicht, indem eine Kostenfunktion des Dithering-Algorithmus verwendet wird.

7. Verfahren nach Anspruch 6, wobei im Entscheidungsschritt, ob ein Schuß auf eine gegebene Gitterposition plaziert werden soll, eine entsprechende Entscheidung hinsichtlich der Gitterpositionen in der Nachbarschaft der gegebenen Gitterposition berücksichtigt wird.

8. Verfahren nach einem der vorhergehenden Ansprüche, das ferner den Schritt aufweist: Unterteilen eines gewünschten Ablationsprofils in mindestens zwei Ablationsteilprofile, Berechnen der Schußdichte jedes der Ablationsteilprofile, Bestimmen einer jeweiligen Gitterweite beruhend auf der jeweiligen berechneten Schußdichte jedes der Ablationsteilprofile.

9. Verfahren nach einem der Ansprüche 5 bis 8, das ferner den Schritt des Sortierens der berechneten plazierten Laserschüsse aufweist.

10. Verfahren nach einem der vorhergehenden Ansprüche, wobei der refraktive Excimer-Laser einen Laserstrahl mit einer festen Fleckgröße zwischen 0,5 mm und 3,5 mm Durchmesser, vorzugsweise mit einer festen Fleckgröße zwischen 1,0 und 2,0 mm Durchmesser liefert.

11. Vorrichtung zur Berechnung einer Laserschußdatei zur Verwendung in einem refraktiven Excimer-Laser vorzugsweise zur Durchführung einer refraktiven Laserbehandlung eines Auges oder zur Herstellung einer kundenspezifischen Kontaktlinse oder einer Intraokularlinse, die aufweist: eine Einrichtung zum Bereitstellen von Informationen bezüglich eines gewünschten Ablationsprofils, eine Einrichtung zur Berechnung einer Schußdichte zum Erhalten des gewünschten Ablationsprofils, wobei die Schußdichte die Anzahl der Laserschüsse ist, die auf einen Teil einer Behandlungszone angewendet werden sollen, eine Einrichtung zur Bestimmung einer Gitterweite eines Gitters, das zum Plazieren von Laserschüssen des Excimer-Lasers auf Gitterpositionen verwendet wird, wobei die Gitterweite der Abstand zwischen zwei Nachbargitterpunkten des Gitters ist und wobei die Gitterweite beruhend auf der berechneten Schußdichte des gewünschten Ablationsprofils bestimmt wird, zur Berechnung der Laserschußdatei mittels eines Dithering-Algorithmus, wobei die Gitterweite so bestimmt wird, daß innerhalb einer Behandlungszone eine minimale Anzahl von Gitterpositionen einen Laserschuß in Teilflächen empfängt, wo die berechnete Schußdichte niedrig ist, und/oder daß eine maximale Anzahl von Gitterpositionen einen Laserschuß in Teilflächen empfängt, wo die berechnete Schußdichte hoch ist.

12. Vorrichtung nach Anspruch 11, wobei innerhalb der Behandlungszone die minimale Anzahl von Gitterpositionen in Teilflächen, wo die berechnete Schußdichte niedrig ist, mindestens 4 Prozent, vorzugsweise mindestens 10 Prozent und besonders bevorzugt mindestens 20 Prozent der Gitterpositionen in den Teilflächen beträgt, wo die berechnete Schußdichte niedrig ist.

13. Vorrichtung nach Anspruch 11 oder 12, wobei innerhalb der Behandlungszone die maximale Anzahl von Gitterpositionen in Teilflächen, wo die berechnete Schußdichte hoch ist, nicht mehr als 96 Prozent, vorzugsweise nicht mehr als 90 Prozent und besonders bevorzugt nicht mehr als 80 Prozent der Gitterpösitionen in den Teilflächen beträgt, wo die berechnete Schußdichte hoch ist.

14. Vorrichtung nach einem der Ansprüche 11 bis 13, wobei die Gitterweite G mittels der Gleichung bestimmt wird

$$G = \sqrt{V_{Shot} * D_{max}(x, y) / z_{max}(x, y)} \ ,$$

wobei

$V_{Shot}$ das Ablationsvolumen eines einzelnen Laserschusses ist,
$D_{max}(x, y)$ die lokale maximale Schußdichte an einer Gitterposition P(x,y) ist,
$z_{max}(x, y)$ der Maximalwert des Ablationsprofils an der Gitterposition P(x,y) ist.

15. Vorrichtung nach einem der Ansprüche 11 bis 14, die ferner eine Einrichtung zur Berechnung der Plazierung der Laserschüsse des Excimer-Lasers auf Gitterpositionen mittels des Dithering-Algorithmus aufweist.

**16.** Vorrichtung nach Anspruch 15, die ferner eine Einrichtung aufweist, um für jede Gitterposition zu entscheiden, ob ein Laserschuß plaziert werden soll oder nicht, indem eine Kostenfunktion des Dithering-Algorithmus verwendet wird.

**17.** Vorrichtung nach Anspruch 16, wobei die Einrichtung zum Entscheiden, ob ein Schuß auf eine gegebene Gitterposition plaziert werden soll, Informationen über eine entsprechende Entscheidung hinsichtlich der Gitterpositionen in der Nachbarschaft der gegebenen Gitterposition empfängt.

**18.** Vorrichtung nach einem der Ansprüche 11 bis 17, die ferner eine Einrichtung zum Unterteilen eines gewünschten Ablationsprofils in mindestens zwei Ablationsteilprofile und zum Berechnen der Schußdichte jedes der Ablationsteilprofile aufweist, wobei die Einrichtung zum Bestimmen einer Gitterweite die jeweilige berechnete Schußdichte jedes der Ablationsteilprofile empfängt und die jeweilige Gitterweite bestimmt.

**19.** Vorrichtung nach einem der Ansprüche 15 bis 18, die ferner eine Einrichtung zum Sortieren der berechneten plazierten Laserschüsse aufweist.

**20.** Vorrichtung nach einem der Ansprüche 11 bis 19, wobei der refraktive Excimer-Laser einen Laserstrahl mit einer festen Fleckgröße zwischen 0,5 mm und 3,5 mm Durchmesser, vorzugsweise mit einer festen Fleckgröße zwischen 1,0 und 2,0 mm Durchmesser liefert.


## Revendications

**1.** Procédé de calcul d'un fichier de tir laser à utiliser dans un laser à excimères de préférence pour effectuer un traitement laser réfractif d'un oeil ou pour produire une lentille de contact personnalisée ou une lentille intraoculaire comprenant les étapes d'utilisation d'un moyen pour fournir des informations concernant un profil d'ablation souhaité, d'utilisation d'un moyen pour calculer une densité de tir pour obtenir le profil d'ablation souhaité, où la densité de tir est le nombre de tirs laser à appliquer à une partie d'une zone de traitement, d'utilisation d'un moyen pour déterminer une largeur de grille d'une grille utilisée pour placer des tirs laser du laser à excimères sur les positions de grille, où la largeur de grille est la distance entre deux points de grille voisins de la grille et où la largeur de grille est déterminée à partir de la densité de tir calculée du profil d'ablation souhaité pour calculer ledit fichier de tir laser en utilisant un algorithme de tramage, où la largeur de grille est déterminée de telle sorte que dans une zone de traitement, un nombre minimal de positions de grille reçoive un tir laser dans les sous-zones où la densité de tir calculée est faible et/ou de telle sorte qu'un nombre maximal de positions de grille reçoive un tir laser dans des sous-zones où la densité de tir calculée est élevée.

**2.** Procédé selon la revendication 1, dans lequel dans la zone de traitement, le nombre minimal de positions de grille dans les sous-zones où la densité de tir calculée est faible est d'au moins 4 pour cent, de préférence au moins 10 pour cent et de manière davantage préférée au moins 20 pour cent de positions de grille dans lesdites sous-zones où la densité de tir calculée est faible.

**3.** Procédé selon la revendication 1 ou 2, dans lequel dans la zone de traitement, le nombre maximal de positions de grille dans les sous-zones où la densité de tir calculée est élevée est non supérieure à 96 pour cent, de préférence non supérieure à 90 pour cent et de manière davantage préférée non supérieure à 80 pour cent de positions de grille dans lesdites sous-zones où la densité de tir calculée est élevée.

**4.** Procédé selon l'une quelconque des revendications précédentes, dans lequel la largeur de grille G est déterminée à l'aide de l'équation :

$$G = \sqrt{V_{Tir} * D_{\max}(x, y) / z_{\max}(x, y)}$$

dans laquelle

$V_{Tir}$ est le volume d'ablation d'un tir laser individuel,
$D_{\max}(x,y)$ est la densité de tir maximale locale à une position de grille P(x,y),

$Z_{max}(x,y)$ est la valeur maximale du profil d'ablation à la position de grille P(x,y).

5. Procédé selon l'une quelconque des revendications précédentes, comprenant en outre l'étape de calcul du placement des tirs laser du lacer à excimères sur des positions de grille à l'aide dudit algorithme de tramage.

6. Procédé selon la revendication 5, comprenant en outre l'étape consistant à décider pour chaque position de grille s'il faut placer un tir laser ou non en utilisant une fonction de coût de l'algorithme de tramage.

7. Procédé selon la revendication 6, dans lequel dans l'étape consistant à décider s'il faut placer un tir sur une position de grille donnée ou non, une décision correspondante concernant les positions de grille dans le voisinage de la position de grille donnée est prise en compte.

8. Procédé selon l'une quelconque des revendications précédentes, comprenant en outre l'étape de division d'un profil d'ablation souhaité en au moins deux sous-profils d'ablation, de calcul de la densité de tir de chacun desdits sous-profils d'ablation, de détermination d'une largeur de grille respective à partir de la densité de tir calculée respective de chacun des sous-profils d'ablation.

9. Procédé selon l'une quelconque des revendications 5 à 8, comprenant en outre l'étape consistant à trier les tirs laser placés calculés.

10. Procédé selon l'une quelconque des revendications précédentes, dans lequel le laser à excimères réfractif fournit un faisceau laser à une taille de point fixée entre 0,5 mm et 3,5 mm de diamètre, de préférence à une taille de point fixée entre 1,0 et 2,0 mm de diamètre.

11. Appareil pour calculer un fichier de tir laser à utiliser dans un laser à excimères réfractif de préférence pour effectuer un traitement laser réfractif d'un oeil ou pour produire une lentille de contact personnalisée ou une lentille intraoculaire comprenant un moyen pour fournir des informations concernant un profil d'ablation souhaité, un moyen pour calculer une densité de tir pour obtenir le profil d'ablation souhaité, où la densité de tir est le nombre de tirs laser à appliquer à une partie d'une zone de traitement, un moyen pour déterminer une largeur de grille d'une grille utilisée pour placer des tirs laser du laser à excimères sur des positions de grille, où la largeur de grille est la distance entre deux points de grille voisins de la grille et où la largeur de grille est déterminée à partir de la densité de tir calculée du profil d'ablation souhaité pour calculer ledit fichier de tir laser en utilisant un algorithme de tramage, où la largeur de grille est déterminée de telle sorte que dans une zone de traitement, un nombre minimal de positions de grille reçoive un tir laser dans des sous-zones où la densité de tir calculée est faible et/ou de telle sorte qu'un nombre maximal de positions de grille reçoive un tir laser dans des sous-zones où la densité de tir calculée est élevée.

12. Appareil selon la revendication 11, dans lequel dans la zone de traitement, le nombre minimal de positions de grille dans les sous-zones où la densité de tir calculée est faible est d'au moins 4 pour cent, de préférence au moins 10 pour cent et de manière davantage préférée au moins 20 pour cent de positions de grille dans lesdites sous-zones où la densité de tir calculée est faible.

13. Appareil selon la revendication 11 ou 12, dans lequel dans la zone de traitement, le nombre maximal de positions de grille dans les sous-zones où la densité de tir calculée est élevée est non supérieure à 96 pour cent, de préférence non supérieure à 90 pour cent et de manière davantage préférée non supérieure à 80 pour cent de positions de grille dans lesdites sous-zones où la densité de tir calculée est élevée.

14. Appareil selon l'une quelconque des revendications 11 à 13, dans lequel la largeur de grille G est déterminée à l'aide de l'équation :

$$G = \sqrt{V_{Tir} * D_{max}(x, y) / z_{max}(x, y)}$$

dans laquelle

$V_{Tir}$ est le volume d'ablation d'un tir laser individuel,
$D_{max}(x,y)$ est la densité de tir maximale locale à une position de grille P(x,y).

$Z_{max}(x,y)$ est la valeur maximale du profil d'ablation à la position de grille $P(x,y)$.

**15.** Appareil selon l'une quelconque des revendications 11 à 14, comprenant en outre un moyen pour calculer le placement des tirs laser du laser à excimères sur des positions de grille à l'aide dudit algorithme de tramage.

**16.** Appareil selon la revendication 15, comprenant en outre un moyen pour décider pour chaque position de grille s'il faut placer un tir laser ou non en utilisant une fonction de coût de l'algorithme de tramage.

**17.** Appareil selon la revendication 16, dans lequel le moyen pour décider s'il faut placer un tir sur une position de grille donnée reçoit les informations d'une décision correspondante concernant les positions de grille dans le voisinage de la position de grille donnée.

**18.** Appareil selon l'une quelconque des revendications 11 à 17, comprenant en outre un moyen pour diviser un profile d'ablation souhaité en au moins deux sous-profils d'ablation, calculer la densité de tir de chacun desdits sous-profils d'ablation, où ledit moyen pour déterminer une largeur de grille reçoit la densité de tir calculée respective de chacun des sous-profils d'ablation et détermine la largeur de grille respective.

**19.** Appareil selon l'une quelconque des revendications 15 à 18, comprenant en outre un moyen pour trier les tirs laser placés calculés.

**20.** Appareil selon l'une quelconque des revendications 11 à 19, dans lequel le laser à excimères réfractif fournit un faisceau laser à une taille de point fixée entre 0,5 mm et 3,5 mm de diamètre, de préférence à une taille de point fixée entre 1,0 et 2,0 mm de diamètre.

Figure 1A

Figure 1B

Figure 1C

Figure 2A

Figure 2B.

Figure 2C

Figure 3

| | | | | | | |
|---|---|---|---|---|---|---|
| | | | | | | |
| | | | | | | |
| | | | | | | |
| 2.667 | 4.000 | 8.000 | Active position | | | |
| 2.530 | 3.578 | 5.657 | 8.000 | 5.657 | 3.578 | 2.530 |
| - | 2.828 | 3.578 | 4.000 | 3.578 | 2.828 | - |
| - | - | 2.530 | 2.667 | 2.530 | - | - |

## Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6090100 A
- US 6271936 B1
- US 2003023233 A